(19) Europäisches Patentamt
European Patent Office
Office européen des brevets

(11) Veröffentlichungsnummer : **0 386 440 B1**

(12) # EUROPÄISCHE PATENTSCHRIFT

(45) Veröffentlichungstag der Patentschrift :
**05.08.92 Patentblatt 92/32**

(51) Int. Cl.$^5$ : **A61K 9/54,** A61K 31/44

(21) Anmeldenummer : **90101675.8**

(22) Anmeldetag : **28.01.90**

(54) **Arzneimittel mit kontrollierter Wirkstoffabgabe.**

Die Akte enthält technische Angaben, die nach dem Eingang der Anmeldung eingereicht wurden und die nicht in dieserPatentschrift enthalten sind.

(30) Priorität : **11.02.89 DE 3904093**

(43) Veröffentlichungstag der Anmeldung :
**12.09.90 Patentblatt 90/37**

(45) Bekanntmachung des Hinweises auf die Patenterteilung :
**05.08.92 Patentblatt 92/32**

(84) Benannte Vertragsstaaten :
**AT BE CH DE DK ES FR GB GR IT LI LU NL SE**

(56) Entgegenhaltungen :
**EP-A- 0 210 540**
**EP-A- 0 250 374**
**EP-A- 0 305 918**
**EP-A- 0 315 414**

(73) Patentinhaber : **BAYER AG**
**W-5090 Leverkusen 1 Bayerwerk (DE)**

(72) Erfinder : **Bücheler, Manfred, Dipl.-Ing.**
**Lorkenhoehe 49**
**W-5064 Overath 2 (DE)**
Erfinder : **Ohm, Andreas, Dr.**
**Am Roettgen 42**
**W-4040 Neuss 1 (DE)**
Erfinder : **Rupp, Roland, Dr.**
**Solinger Strasse 18**
**W-5653 Leichlingen 2 (DE)**
Erfinder : **Schmoll, Josef, Dl.**
**Irlenweg 6**
**W-5632 Wermelskirchen 2 (DE)**
Erfinder : **Wollenschläger, Axel, Dr.**
**Walter-Flex-Strasse 19**
**W-5090 Leverkusen (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Wirkstoffabgabesystem in Pellet-Form, mit der die Wirkstofffreisetzung zeitlich gesteuert, in gepulster Form und auch in ihrer jeweiligen Steilheit eingestellt werden kann. Die erfindungsgemäßen Pellets bestehen aus einem wirkstoffhaltigen Kern, der von einem polymerhaltigen Mantel und einer unverdaulichen wasserdurchlässigen Lackschicht umgeben ist.

Der therapeutische Nutzen eines Arzneimittels wird nicht nur von der Art des verwendeten Wirkstoffs bestimmt sondern in hohem Maße von der speziellen galenischen Darreichungsform. Bei vielen Arzneimitteln läßt sich durch Optimierung der Zubereitungsform die Wirkungseffektivität steigern, die unerwünschten Nebenwirkungen verringern und die Behandlungssicherheit erhöhen bei gleichzeitiger Verbesserung der Patientkompliance. Durch spezielle galenische Zubereitungen kann erreicht werden, daß der Wirkstoff zum richtigen Zeitpunkt und in optimaler Dosierung das Resorptionsorgan erreicht (vergl. K. Heilmann, Therapeutic Systems, Rate-controlled Drug Delivery; Concept and Development; New York (1984) Thieme Stratton).

Es gibt zahlreiche Versuche, auch für schwer lösliche Wirkstoffe, Systeme zu entwickeln, die eine zeitlich und örtlich kontrollierte Freigabe des Wirkstoffs in optimalen Konzentrationen gewährleisten sollen. So wurde z. B. versucht, über osmotische Mechanismen eine zeitlich gesteuerte kontinuierliche oder diskontinuierliche Freisetzung von Wirkstoffen durch Herauspressen des Wirkstoffs aus einer vorgegebenen Öffnung zu erreichen (vergl. DE-A1 3 715 227).

Die Möglichkeit einer zeitlich gesteuerten kontinuierlichen Freisetzung wurde auch versucht mit Erosionssystemen oder mit lackierten Systemen, wobei die Lackschicht eine teilweise durchlässige Membran mit retardierender Wirkung darstellt (vergl. WO 88/00046). Nachteilig für solche kontinuierlich freisetzenden Systeme ist jedoch die häufig mit der Zeit abnehmende Wirkstoffreisetzung und bei nicht erodierenden Matrixsystemen und lackierten Systemen der Effekt, daß der Wirkstoff häufig nicht quantitativ abgegeben wird. Als weitere Nachteile dieser Systeme sind zu nennen, daß z. B. die osmotischen Systeme nur sehr kompliziert und aufwendig herzustellen sind und häufig die bekannten Nachteile von Einzeldosisarzneiformen auftreten wie z. B. dose dumping und breite Variabilität der Passage-Zeit im Körper in Abhängigkeit von Individuum und Ernährung. Darüberhinaus lassen sich mit diesen Systemen mehrfach gepulste Freisetzungsstöße praktisch nicht realisieren.

Zur Vermeidung des Nachteils der mit der Zeit abnehmenden Wirkstofffreisetzung, wurde auch versucht, mehrschichtige Tabletten mit unterschiedlicher Konzentration an aktiver Substanz in den verschiedenen Schichten herzustellen (DE-OS 1 767 765) oder die Konzentration des Wirkstoffs von der Hülle zum Kern hin mit ansteigendem Konzentrationsgradienten zu erhöhen (vergl. DE-PS 2 651 176). Solche Zubereitungsformen sind nur mit hohem technischen Aufwand herzustellen, ermöglichen nicht beliebig häufige Freisetzungsstöße und unterliegen in Tablettenform in starkem Maße den unterschiedlichen Passage-Zeiten und Ernähungsgewohnheiten der Patienten.

Zur Vermeidung einiger dieser Nachteile wurden auch Tabletten oder Kapseln mit gesteuerter Wirkstofffreigabe vorgeschlagen, die eine größere Anzahl von kleinen Dosierungseinheiten in Form von Kernen, Perlchen, Granulen oder Pellets enthalten (s. DE-OS 1 617 724 und US-PS 3 247 066). Die dort beschriebenen Perlchen oder Pellets sollen das Medikament über einen Zeitraum von bis zu 12 Stunden freigeben. Durch die Dimension und der Vielzahl der kleinen Perlchen funktioniert die Freigabe weitgehend unabhängig von den verschiedenen physiologischen Bedingungen beim einzelnen Patienten. Die wirkstoffhaltigen Kerne dieser Perlchen oder Pellets sind von einem unverdaulichen, wasserdurchlässigen leicht zerreißenden Film umgeben. Der Kern enthält neben dem Wirkstoff ein in Wasser quellbares Kolloid. Bei Kontakt mit der wasserhaltigen Körperflüssigkeit beginnt der Kern das Wasser zu absorbieren und zu quellen, was letztlich nach einer bestimmten Zeit zum Zerplatzen des Filmüberzugs und zur verstärkten Freisetzung bzw. Absorption des freigewordenen Wirkstoffs führt. Der Beginn der Haupt-Absorption liegt hierbei einige Zeit nach dem Zeitpunkt der Einnahme und kann über Art und Dicke des Filmüberzugs und Art und Menge der Quellstoffe weitgehend gesteuert werden. Als bevorzugtes quellendes Kolloid is Gelatine genannt und als Material für die Lackhülle ist vorzugsweise Ethylcellulose gennant. Das Ziel dieser Anmeldung ist eine gesteuerte Freigabe des Wirkstoffs unabhängig vom pH-Wert der verschiedenen Körperflüssigkeiten, z. B. dem sauren Magensaft und der alkalischen Intestinalflüssigkeit.

Ein Nachteil dieses Systems besteht darin, daß schon vor dem Zerreißen der Lackschicht Wirkstoffanteile durch Diffusion freigesetzt werden können. Weiterhin lassen sich nach diesem System zwar Verzögerungszeiten erzielen aber die erreichbare Steilheit der anschließenden Wirkstoffreisetzung nach Platzen der Hülle ist nicht optimal. Insbesondere bei Wirkstoffen mit einem sättigbaren "first pass effect" ist eine steile Wirkstofffreisetzung wünschenswert, um gute Bioverfügbarkeiten bei geringer Belastung des Organismus mit Wirkstoff zu erzielen. Ein weiterer Nachteil liegt darin, daß in diesen Kern-Lack-Pellets der Wirkstoff schon sehr früh mit dem wäßrigen Freisetzungsmedium benetzt wird. Das kann dazu führen, daß bereits Stunden vor Erreichen

der Lag-Zeit unerwünschte Reaktionen der Körperflüssigkeit mit dem Wirkstoff stattfinden wie z. B. Rekristallisation und damit Veränderung der Lösungs- und Absorptionseigenschaften oder chemische Veränderungen des Wirkstoffs.

Eine Variante dieser "zerplatzenden" Arzneiformen wird auch in EP-A 210 540 beschrieben. Das dort als "time-controlled explosion system" bezeichnete Prinzip zur kontrollierten Wirkstofffreisetzung zeichnet sich dadurch aus, daß direkt unter einer wasserdurchlässigen Lackschicht oder Membran, die einen Zusatz wie Talk, PEG, Silicon etc. enthalten, eine hydrophile Schicht mit starken Quellmitteln, wie z.B. Hydroxypropylcellulose, oder Sprengmitteln liegt. Diese Schichten sind entweder wirkstofffrei (vergl. Figur 1 und 2) oder enthalten bereits Wirkstoff (vergl. Figur 3 und 4). Durch den direkten Kontakt der Sprengmittel bzw. der Quellschicht mit der äußeren Membran beginnt in diesem System der Aufbau des Explosionsdrucks unmittelbar nachdem das Wasser durch die äußere Membran diffundiert ist. Durch diesen Aufbau ist es schwierig, Lag-Zeiten von mehreren Stunden sicherzustellen. Die Freisetzungsdaten der Tabelle 1 zeigen für verschiedene Schichten der Ethylcellulosemembran unterschiedliche Lag-Zeiten, die selbst bei einer sehr dicken Ethylcellulose-Schicht, die ca. 30 % des Gewichts beträgt (sample C) nur weniger als 4 Stunden beträgt. Dies zeigt auch die Publikation von Satoshi Ueda et al. in Proceed Intern. Symp. Control. Rel. Bioact. Mater., 15 (1988) Controlled Release Society, Inc., Nr. 254, Seiten 450-451. Die dort wiedergegebenen Ergebnisse, insbesondere Figur 3 zeigt, daß die maximal erreichbare Lag-Zeit bei diesem System unter 5 Stunden liegt, wobei die gewünschte Steilheit der Freisetzungskurve nach diesem Zeitraum bereits nicht mehr erreicht werden kann.

Demgegenüber weist das erfindungsgemäße Wirkstoffabgabesystem eine Reihe von Vorteilen auf die dadurch erreicht werden, daß dem inneren Mantel, der aus Hydroxypropylcellulose von Typ M oder H besteht, ein hydrophober Zusatz beigemengt wird. Durch Kombination der erfindungsgemäßen Pellets, z. B. durch die Verwendung unterschiedlicher Pellet-Kollektive, sind beliebige Freisetzungsprofile auch über einen Zeitraum von mehr als 12 Stunden realisierbar. Von besonderem Vorteil sind die bisher nicht erreichbaren langen Verzögerungszeiten, nach denen wahlweise dann sehr steile Freisetzungen oder auch weniger steile Freisetzungen programmiert werden können. Durch diese gepulste, steuerbare Wirkstofffreisetzung in beliebigen Intervallen wird der erhöhte Wirkstoffabbau bei sättigbarem first pass effect überwunden. Dies ermöglicht eine Dosisreduktion wegen der geringeren Metabolisierung und vermeidet eine unnötige Belastung der metabolisierenden Organe. Durch geeignete Kombination unterschiedlich freisetzender Pellet-Kollektive ist eine Anpassung der Wirkstofffreisetzung an den tageszeitlichen Bedarf bzw. an den biologischen Rhythmus möglich. Ebenso können die Wirkstoffe nach diesem System in bestimmten Resorptionsabschnitten (Resorptionsfenster) in unterschiedlichen Bereichen des Gastrointestinaltraktes bereitgestellt werden. Ein vorzeitiger enzymatischer, bakterieller oder chemischer Abbau des Wirkstoffs wird hierdurch ausgeschlossen. Eine unnötige Irritation in den Resorptionsorganen wird vermieden. Gleichzeitig verringert sich das Risiko durch zeitlich falsche Einnahme durch den Patienten. Die Unabhängigkeit der gewünschten Verzögerungszeit von unterschiedlichen pH-Werten und Eßgewohnheiten erhöht die Arzneimittelsicherheit und die Effektivität der Behandlung.

Gegenstand der vorliegenden Erfindung sind neue Arzneimittel mit kontrollierter Wirkstoffabgabe enthaltend mindestens ein Pelletkollektiv, dadurch gekennzeichnet, daß die Pellets aufgebaut sind aus

a) einen Kern, der als Wirkstoff eine Verbindung der allgemeinen Formel

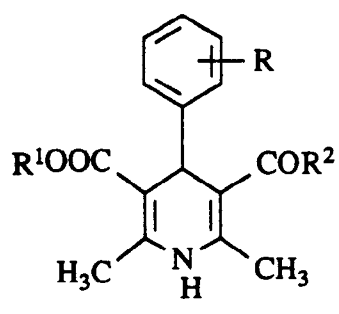

in welcher

R für Nitro oder die Gruppe

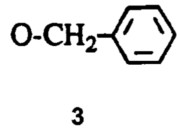

in ortho- oder meta-Position steht,

$R^1$ für Alkyl mit 1 - 4 C-Atomen steht, das gegebenenfalls durch ein Sauerstoff in der Kette unterbrochen ist und

$R^2$ für Alkoxy mit 1 - 4 C-Atomen oder für den Rest

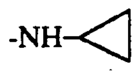

steht, oder

die Verbindung 3-(4-Fluorphenylsulfonamido) 1.2.3.4-tetrahydro-9-carbazol-propanoylsäure oder die Verbindung 2-[4-[4-(2-Pyrimidinyl)-1-piperazinyl]butyl]-1.2-benzisothiazol-3(2H)-on-1,1-dioxid, monohydrochlorid, enthält,

ein Intensivsprengmittel aus der Gruppe quervernetzte Natriumcarboxymethylcellulose oder Natriumstärkeglycolat (sodium starch glycolat; NF XVI), als Netzmittel Natriumlaurylsulfat und als Bindemittel PVP-25 enthält, und

b) einer freisetzungskontrollierenden Doppelschicht, bestehend aus

b1) einer äußeren unverdaulichen wasserdurchlässigen Lackschicht, die im wesentlichen aus Acrylharzen auf Basis von Poly(meth)acrylsäureestern mit neutralem Charakter (NE-Typ) oder mit einem geringen Gehalt an quartären Ammoniumgruppen (R-Typ) besteht, wobei der NE-Typ aus Copoly(meth)acrylsäureestern mit dem Strukturelement

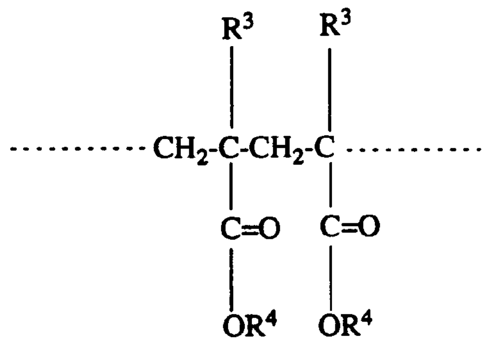

besteht, wobei

$R^3$ für H oder $CH_3$ steht, und

$R^4$ für $CH_3$ oder $C_2H_5$ steht

und ein mittleres Molekulargewicht von ca. 800.000 hat, und

der R-Typ sich hiervon dadurch unterscheidet, daß $R^4$, in einem molaren Verhältnis von 1:20 bis 1:40, für die Gruppe

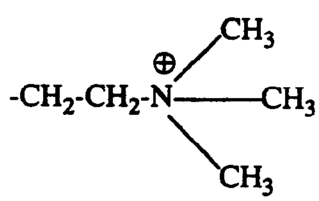

steht, und ein mittleres Molekulargewicht von ca. 150.000 besitzt,

oder

aus Ethylcellulose besteht, und wobei

diese äußere Lackschicht gegebenenfalls als zusätzliche Hilfsstoffe Trennmittel wie Magnesiumstearat oder Calciumstearat enthält und

gegebenenfalls zusätzlich übliche Weichmacher wie Polyethylenglykol 20.000, Dialkyl-(1-4 CA)-diphthalat, Glycerintriacetat oder Zitronensäureester wie Triethylcitrat enthält, und

b2) einem inneren Mantel, der die Migration des Wassers in Richtung Kern kontrolliert, der 10 bis zu 40 % aus Hydroxypropylcellulose vom Typ M oder H besteht (HPC-M oder HPC-H) und zu 60 % bis 90 % aus

einem hydrophoben Zusatz, vorzugsweise Calciumstearat oder hydriertem Rizinusöl besteht.

Von besonderem Interesse sind Pellets mit einem Teilchendurchmesser von 0,8 bis 3,5 mm, vorzugsweise von 1,0 bis 3 mm, insbesondere 1,5 bis 2,5 mm mit einem Gewicht von 0,5 - 20 mg, insbesondere 2 - 10 mg pro Pellet. Der Gewichtsanteil des wirkstoffhaltigen Kerns beträgt 20 - 50 %, insbesondere 25 - 40 % des Gesamtgewichts des Pellets und der Kern hat vorzugsweise einen Durchmesser von 0,5 - 1,5 mm. Der Gehalt der Dihydropyridinwirkstoffe im Kern beträgt vorzugsweise 40 - 90 %, insbesondere 60 - 85 % des Kerngewichts.

Die äußere Lackschicht hat vorzugsweise eine Dicke von bis zu 0,3 mm, insbesondere bis zu 0,2 mm. Das Gewicht der Lackschicht beträgt bis zu 50 %, insbesondere bis zu 35 % bezogen auf das Gesamtgewicht des Pellets.

Die migrationskontrollierende Mantelschicht hat eine Dicke von ca. 0,1 bis 0,5 mm, inbesondere von 0,2 bis 0,4 mm. Das Gewicht dieser Mantelschicht beträgt ca. 25 - 65 %, insbesondere 30 - 55 % des Pelletgewichts.

Der Anteil der Hydroxypropylcellulose im Mantel beträgt ca. 10 bis 40 %, insbesondere 15 bis 30 % des gesamten Mantelgewichts und der Anteil des lipophilen Bestandteils des Mantels insbesondere 70 bis 85 % des Gewichts der Mantelschicht.

Der Kern enthält die Dihydropyridinwirkstoffe in einem Gewichtsverhältnis von 40 bis 90 %, insbesondere 50 bis 85 % bezogen auf das Kerngewicht. Der Anteil des Intensivsprengmittels beträgt ca. 3 bis 30 %, insbesondere 5 bis 20 % des Kerngewichts. Der Netzmittelanteil (Natriumlaurylsulfat) am Kerngewicht beträgt 0,5 bis 5 %, insbesondere 1 bis 3 % und der Bindemittelanteil (PVP-25) beträgt ca. 3 bis 25 %, insbesondere 5 bis 20 % des Kerngewichts.

Besonders geeignet ist die erfindungsgemäße Formulierung für die Wirkstoffe Nifedipin, Nimodipin, Nisoldipin und 4-(2-Benzyloxy-phenyl)-1,4-dihydro-2,6-dimethylpyridin-3-carbonsäuremethylester-5-carbonsäurecyclopropylamid/sowohl in Racemform als auch als Enantiomer und für 3-(4-Fluorphenylsulfonamido)-1,2,3,4-tetrahydro-9-carbazol-propanoylsäure und für 2-[4-[4-(2-pyrymidinyl)-1-piperazinyl]butyl]-1,2-benzisothiazol-3(2H)-on-1,1-dioxid, monohydrochlorid.

Der Kern enthält vorzugsweise als Intensivsprengmittel quervernetzte Natriumcarboxymethylcellulose (Crosscarmellosesodium USP XXI N.F. 16 Typ A), als Netzmittel Natriumlaurylsulfat und als Bindemittel Polyvinylpyrrolidon 25 (PVP 25).

Der Mantel enthält Hydroxypropylcellulose in Form von HPC-M oder HPC-H und als lipophilen Zusatz vorzugsweise Calciumstearat.

Der Lack enthält vorzugsweise Poly(meth)acrylsäureester der Typen RS, RL und NE30D, insbesondere die unter dem Warenzeichen Eudragit RS(R), Eudragit RL(R) und Eudragit NE30D(R) bekannten Polymere, die von der Firma Roehm Tech. Inc., USA,vertrieben werden.

Durch die erfindungsgemäße Kombination einer wasserdurchlässigen aber unlöslichen Lackschicht mit einer wirkstofffreien Mantelschicht, die durch ein bestimmtes Gemisch von hydrophoben und hydrophilen Bestandteilen die Migration des Wassers zum Kern kontrolliert, ohne zu einem Platzen der Lackhülle zu führen, mit dem wirkstoffhaltigen Kern, der den Wirkstoff und gleichzeitig Intensiv-Sprengmittel enthält, läßt sich die Freisetzung des Wirkstoffs aus den jeweiligen Pellets für einen Zeitraum von mehr als 12 Stunden verzögern. Durch Kombination unterschiedlicher Pellet-Kollektive ist eine gepulste Freisetzung erreichbar, die bei einmal täglicher Einnahme an den unterschiedlichen Wirkstoffbedarf des Tagesrhythmus des Patienten angepaßt werden kann. Dies ist vor allem bei der Langzeittherapie z. B. bei Bluthochdruck von Bedeutung. So läßt sich z. B. die Phase des niedrigen Blutdrucks während der Nacht mit einer entsprechenden Lag-Phase koordinieren, so daß der Freisetzungsschub mit dem Anstieg des Blutdrucks in den frühen Morgenstunden zusammenfällt.

Die Steilheit der Freisetzung läßt sich über den Gehalt des Sprengmittelanteils im Kern steuern. Auch nach Verzögerungszeiten von mehr als 6 Stunden lassen sich sehr enge Freisetzungsintervalle erreichen.

Das Normierte Freisetzungsintervall (NFI) sei folgendermaßen definiert: NFI = (t80 - t20/t50, wobei t80 der Zeitpunkt ist, zudem 80 % des Wirkstoffs freigesetzt sind und analog t20 bzw. t50 der Zeitpunkt ist, an dem 20 bzw. 50 % des Wirkstoffs freigesetzt sind. Durch die erfindungsgemäßen Pellets kann auch nach einer Verzögerungszeit von mehr als 6 Stunden ein NFI von weniger als 0,25, insbesondere 0,20 erzielt werden.

Ein weiteres Charakteristikum der erfindungsgemäßen Pellets ist, daß bis zu 90 % der Lag-Time weniger als 5 % des Wirkstoffs freigesetzt werden. Dies ermöglicht eine sehr exakte Einstellung der retardierten bzw. der pulsierenden Freisetzungsstöße.

Selbstverständlich können mittels der erfindungsgemäßen Pellets auch langsamere Freisetzungen, d. h. größere NFI-Werte erreicht werden, z. B. durch Verringerung des Sprengmittelanteils im Kern. Ebenso lassen sich gleichförmige (nicht gepulste) Freisetzungen verwirklichen z. B. durch die Verwendung einer größeren Anzahl von Pellets, deren Lag-Zeiten sich über den gesamten Freisetzungszeitraum gleichmäßig verteilen.

Überraschenderweise lassen sich durch das synergistische Zusammenwirken der Lackschicht und der

Mantelschicht sehr lange Verzögerungszeiten bei nur geringen Schichten des Placeboanteils des kleinen Pellets ermöglichen. Nach den bisher bekannten Zubereitungen wurde die Lag-Zeit bzw. der Vorzögerungszeitraum nur über 1 oder 2 Parameter gesteuert, z. B. nur durch Erosion oder wie bei EP-A 210 540 einerseits durch die wasserdurchlässige Lackschicht und andererseits durch Art und Menge der Sprengmittel im benachbarten Mantelbereich. Durch das erfindungsgemäße System wird durch die wirkstofffreie Mantelschicht ein weiterer Kontrollparameter für die Verzögerungszeit zur Verfügung gestellt. Lack- und Mantelschicht ermöglichen eine effektive Kontrolle der Migrationsgeschwindigkeit des Wassers zum Kern hin. Sie gewährleisten ein einheitliches Vordringen der Wasserfront und damit eine sehr exakte Steuerung der Lag-Zeiten mit steilen Freisetzungen. Damit wird der Fachmann erstmals in die Lage versetzt, die bekannten Vorteile der Pellets mit einem Teilchendurchmesser von unter 3 mm einzusetzen für Retardformulierungen bzw. für langwirkende, Tagesrhythmus angepaßte Formulierungen mit gepulster Freisetzung.

Für die erfindungsgemäßen Pellets lassen sich ohne weiteres auch übliche galenische Maßnahmen anwenden. So kann z. B. die Lack- und/oder Mantelschicht zum Zwecke des Lichtschutzes oder zur besseren Unterscheidbarkeit mit üblichen Arzneifarbstoffen angefärbt werden, mit Salzen, die die Osmose oder den pH-Wert beeinflussen oder mit Geschmacksverbesserern versetzt werden.

Die Herstellung der erfindungsgemäßen Pellets erfolgt nach üblichen Methoden. Die Herstellung des Kerns kann z.B. durch Rollgranulation, Mischgranulation, Tablettierung, Wirbelschichtgranulation oder Wirbelschichtsprühgranulation in kontinuierlicher oder diskontinuierlicher Arbeitsweise erfolgen. Besonders bevorzugt sind Methoden der Mischgranulation und der Rollgranulation z. B. Teller-, Trommel- und Rotorgranulation. Die Bestandteile des Kernes (Wirkstoff, Sprengmittel und Hilfsstoffe) weisen vorzugsweise Partikelgrößen von weniger als 100 µm auf, um eine hohe Sphärizität und Oberflächengüte zu erzielen.

Die Herstellung des Kerns erfolgt z. B. durch Mischen des Wirkstoffs, des Intensivsprengmittels, des Netzmittels und des Bindemittels in einem Mischer, Zugabe von Wasser und/oder organischen Lösungsmitteln wie niederen aliphatischen Alkoholen oder Aceton als Granulierflüssigkeit, 0,5 - 3 Stunden Granulation und anschließender Trocknung bei 30 - 120°C, vorzugsweise 40 - 100°C. Anschließend wird das erhaltene Granulat gesiebt.

Der Auftrag der Mantelschicht auf die Kerne erfolgt ebenfalls nach üblichen Methoden z. B. durch Aufsprühen aus Lösung, Schmelze oder Suspension. Das Verfahren kann durchgeführt werden im mechanischen Mischer, in der Wirbelschicht, auf dem Granulierteller, in der Granuliertrommel oder im Rotorgranulator. Außerdem kann das Mantelmaterial auch pulverförmig unter Zugabe von Granulierflüssigkeit aufgetragen werden z. B. in diskontinuierlicher oder kontinuierlicher Weise in üblichen Apparaturen für die Rollgranulation.

Bei der Herstellung der Mantelschicht wird der Hydrogelbildner Hydroxypropylcellulose (HPC-M oder HPC-H) vorzugsweise mit einer Partikelgröße von weniger als 100 µm, insbesondere weniger als 65 µm eingesetzt.

Der Auftrag des Lackes erfolgt in üblicher Weise z. B. durch Aufsprühen aus organischer Lösung oder aus wäßriger Suspension in Lackierkesseln, rotierenden Trommeln oder Tellern oder in üblichen Coatern. Der Lackauftrag erfolgt vorzugsweise aus wäßriger Dispersion bei erhöhten Temperaturen, bei denen Filmbildung eintritt, vorzugsweise bei 30 - 100°C, insbesondere bei 40 - 80°C.

Bei einem Lackauftrag aus Lösung werden vorzugsweise organische Lösungsmittel aus der Gruppe niedere aliphatische Alkohole wie Ethanol, Methanol, Isopropanol, flüchtige Ketone wie z. B. Aceton oder halogenierte Kohlenwasserstoffe wie z. B. Dichlormethan oder Chloroform eingesetzt.

Die Abfüllung der gemischten oder ungemischten Pellet-Kollektive in Kapseln erfolgt mit üblichen Füll- und Verschließmaschinen. Neben dem Einfüllen der Pellets in Kapseln ist es auch möglich, die Pellets in einen Pressling einzubringen.

Ein bevorzugtes Verfahren zur Herstellung der erfindungsgemäßen Pellets besteht darin, daß man

a) den Kern herstellt, indem man den Wirkstoff, das Intensivsprengmittel, das Netzmittel und das Bindemittel mischt und dann unter Zugabe von Wasser und/oder organischen Lösungsmitteln wie niederen aliphatischen Alkoholen oder Aceton als Granulierflüssigkeit 0,5 bis 3 Stunden granuliert, anschließend bei 30 bis 120°C trocknet und das erhaltene Granulat siebt, und

b) die Mantelschicht durch Aufsprühen aus Lösung, Schmelze oder Suspension der Hydroxypropylcellulose und des hydrophoben Zusatzes in der Wirbelschicht, auf dem Granulierteller, im mechanischen Mischer oder im Rotorgranulator aufträgt, oder

das Mantelmaterial in Pulverform unter Zugabe von Granulierflüssigkeit in diskontinuierlicher oder kontinuierlicher Weise in üblichen Apparaturen für die Rollgranulation aufträgt, und

c) die Lackbestandteile entweder gelöst in organischen Lösungsmitteln wie niedere aliphatische Alkohole, flüchtige Ketone oder halogenierte Kohlenwasserstoffe aufsprüht, oder

aus wäßriger Suspension in Lackierkesseln, rotierenden Trommeln oder Tellern oder in üblichen Coatern bei Temperaturen zwischen 30 und 100°C aufträgt und thermisch vernetzt.

Die Bestimmung der Freisetzung der erfindungsgemäßen Pellets erfolgt nach der USP Paddle Methode.

Es werden jeweils mit Pellets gefüllte Kapseln eingesetzt, die 30 mg Wirkstoff enthalten. Der Test wird bei 37°C in 4.000 ml Freisetzungsmedium und bei 100 Umdrehungen pro Minute durchgeführt. Das Freisetzungsmedium ist mit einem Puffer (DAB-9; 1:10 verdünnt) auf pH 6,8 eingestellt und enthält zusätzlich 0,25 % Natriumlaurylsulfat und 0.68 % Natriumchlorid.

Die aus den erfindungsgemäßen Pellets herstellbaren Verabreichungsformen wie Kapseln oder Presslinge können nach üblichen Methoden hergestellt werden, wobei auch Kombinationen mit anderen Wirkstoffen möglich sind. Falls sofort wirkende Initialdosen erwünscht sind, können die erfindungsgemäßen Pellets auch mit schnell freisetzenden Formen der obengenannten Dihydropyridine, z. B. mit Kopräzipitaten oder mit nicht lackierten Kernen kombiniert werden.

Die nachfolgenden Ausführungsbeispiele erläutern die erfindungsgemäßen Kern-Mantel-Lack-Pellets (KML-Pellets).

## Ausführungsbeispiele

### Beispiel 1

Herstellung des Kerns

850 g mikronisiertes Nifedipin werden mit 80 g quervernetzter Natriumcarboxymethylcellulose, 50 g PVP-25 und 20 g Natriumlaurylsulfat in einem Intensivmischer gemischt. Anschließend werden 200 ml destilliertes Wasser zugegeben und 3 Stunden bei Raumtemperatur mit abnehmender Drehzahl (3.000 → 400 UpM) granuliert. Die erhaltenen Kerne werden getrocknete bei 80°C und gesiebt (Durchmesser 1,25 - 1,5 mm).

Aufbringung des Mantels

1.000 g dieser Kerne werden in einem Rotorgranulator vorgelegt und kontinuierlich mit einer Mischung aus 510 g Hydroxypropylcellulose-M (entspricht 30 % des Mantelanteils) und 1.190 g hydriertem Rizinusöl (entspricht 70 % des Mantelanteils) und Wasser als Granulierflüssigkeit versetzt und in einem Rotorgranulator (250 UpM) bei Raumtemperatur ummantelt. (Dosisrate des Pulvers 1000 g/h). Die Bettfeuchte wird dabei auf 25 % absolute Feuchte durch Dosierung der Granulierflüssigkeit geregelt.

Lackauftrag

Die ummantelten Kerne (2.700 g) werden in einem Rotorcoater mit einer 10 %igen wäßrigen Dispersion, bestehend aus 50.6 % Eudragit RS, 44,9 % Magnesiumstearat und 4,5 % PEG 20.000, jeweils bezogen auf das Gewicht des Feststoffanteils, bei 60 - 70°C besprüht. Nach 1,5 Stunden erhält man Pellet-Kollektive mit 30 % Lackanteil. Nach 4,0 Stunden erhält man Pellets mit 80 % Lackanteil.

Je nach Dicke der Lackschicht ergeben sich folgende Verzögerungszeiten

| **Menge der Lackschicht bezogen auf das Kerngewicht** | **Verzögerungszeit** |
|---|---|
| 0 % (kein Lack) | 1,5 Stunden |
| 30 % | 4,0 Stunden |
| 80 % | 8,5 Stunden |

### Beispiel 2

Analog Beispiel 1 werden Kern-Mantel-Lackpellets folgender Rezeptur hergestellt:

| | Gewichtsteile: |
|---|---|
| Kern (1 Teil) | |
| Nifedipin | 73 % |
| quervernetzte Natriumcarboxymethylcellulose | 15 % |
| PVP 25 | 10 % |
| Natriumlaurylsulfat | 2 % |
| | |
| Mantel (1,7 Teile) | |
| Hydroxypropylcellulose-M | 30 % |
| Calciumstearat | 70 % |
| | |
| Lack (0,1 - 1,09 Teile) | |
| Eudragit RS | 50,6 % |
| Magnesiumstearat | 44,9 % |
| PEG-20.000 | 4,5 % |

| Kurve | Lackauftragsmenge bezogen auf Kerngewicht = 100 % | Verzögerungszeit |
|---|---|---|
| 1 | 0 % (kein Lack) | 1,0 Stunde |
| 2 | 10 % | 2,0 Stunden |
| 3 | 28 % | 4,0 Stunden |
| 4 | 44 % | 5,6 Stunden |
| 5 | 64 % | 7,0 Stunden |
| 6 | 109 % | 12 Stunden |

Die Freisetzungsraten für die verschieden dicken Lackschichten sind aus Abbildung 1 zu ersehen.

Beispiel 3

Analog Beispiel 1 wurden KML-Pellets folgender Rezeptur hergestellt:

| | | Gewichtsteile |
|---|---|---|
| Kern (1 Teil) | | |
| Nisoldipin | | 73 % |
| quervernetztes Natriumcarboxymethylcellulose | | 15 % |
| PVP 25 | | 10 % |
| Natriumlaurylsulfat | | 2 % |
| Mantel (1,7 Teile) | | |
| Hydroxypropylcellulose-M | | 30 % |
| Calciumstearat | | 70 % |
| Lack (0,11 - 1,07 Teile) | | |
| Eudragit RS | | 50,6 % |
| Magnesiumstearat | | 44,9 % |
| PEG-20.000 | | 4,5 % |

| Kurve | Lackauftragsmenge bezogen auf Kerngewicht = 100 % | Verzögerungszeit |
|---|---|---|
| 1 | 0 % (kein Lack) | 1,0 Stunde |
| 2 | 11 % | 2,0 Stunden |
| 3 | 29 % | 3,0 Stunden |
| 4 | 43 % | 4,0 Stunden |
| 5 | 63 % | 5,5 Stunden |
| 6 | 107 % | 10 Stunden |

Die Freisetzungsraten für die verschieden dicken Lackschichten sind aus Abbildung 2 zu ersehen.

Beispiel 4

Analog Beispiel 1 wurden KML-Pellets folgender Rezeptur hergestellt:

| | | Gewichtsteile |
|---|---|---|
| Kern (1 Teil) | | |
| Nimodipin | | 73 % |
| quervernetzte Natriumcarboxymethylcellulose | | 15 % |
| PVP-25 | | 10 % |
| Natriumlaurylsulfat | | 2 % |
| Mantel (1,3 Teile) | | |

| | |
|---|---|
| Hydroxypropylcellulose-M | 30 % |
| Calciumstearat | 70 % |
| | |
| Lack (0,1 - 1,07 Teile) | |
| Eudragit RS | 50,6 % |
| Magnesiumstearat | 44,9 % |
| PEG-20.000 | 4,5 % |

| Lackauftragsmenge bezogen auf Kerngewicht = 100 % | Verzögerungszeit |
|---|---|
| 0 % | 0,7 Stunden |
| 10 % | 1,5 Stunden |
| 29 % | 3,4 Stunden |
| 48 % | 5,1 Stunden |
| 67 % | 6,5 Stunden |
| 107 % | 10,3 Stunden |

Beispiel 5

Analog Beispiel 1 wurden KML-Pellets folgender Rezeptur hergestellt:

| | Gewichtsteile |
|---|---|
| Kern (1 Teil) | |
| Nisoldipin | 60 % |
| Sodium Starch Glycolat | 23 % |
| PVP-25 | 15 % |
| Natriumlaurylsulfat | 2 % |
| | |
| Mantel (1,2 Teile) | |
| Hydroxypropylcellulose-H | 20 % |
| hydriertes Rizinusöl | 80 % |
| | |
| Lack (0,1 - 1,2 Teile) | |
| Eudragit NE30D | 55 % |
| Magnesiumstearat | 44 % |
| Glycerintriacetat | 1 % |

Beispiel 6

Analog Beispiel 1 wurden KML-Pellets folgender Rezeptur hergestellt:

| | Gewichtsteile |
|---|---|
| Kern (1 Teil) | |
| 4-(2-benzyloxyphenyl)-1,4-dihydro-2,6-dimethylpyridin-(3-carbonsäure-methyl ester)-(5-carbonsäure-cyclopropylamid) | 70 % |
| Sodium Starch Glycolat | 18 % |
| PVP-25 | 10 % |
| Natriumlaurylsulfat | 2 % |
| Mantel (1 Teil) | |
| Hydroxypropylcellulose-H | 30 % |
| Calciumstearat | 70 % |
| Lack (0,1 - 1,2 Teile) | |
| Eudragit NE30D | 50,6 % |
| Magnesiumstearat | 44,9 % |
| PEG-20.000 | 4,5 %. |

## Patentansprüche

### Patentansprüche für folgende Vertragsstaaten : AT,BE,CH,DE,DK,FR,GB,IT,LI,LU,NL,SE

1. Arzneimittel mit kontrollierter Wirkstoffabgabe enthaltend mindestens ein Pellet-Kollektiv, dadurch gekennzeichnet, daß die Pellets aufgebaut sind aus

a) einen Kern, der als Wirkstoff eine Verbindung der allgemeinen Formel

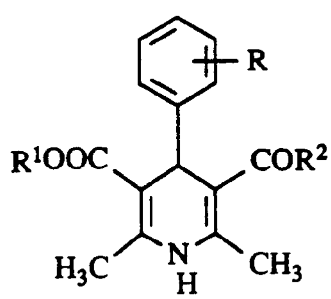

in welcher

R für Nitro oder die Gruppe

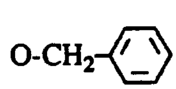

in ortho- oder meta-Position steht,

$R^1$ für Alkyl mit 1 - 4 C-Atomen steht, das gegebenenfalls durch ein Sauerstoff in der Kette unterbrochen ist und

$R^2$ für Alkoxy mit 1 - 4 C-Atomen oder für den Rest

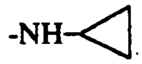

steht, oder

die Verbindung 3-(4-Fluorphenylsulfonamido) - 1.2.3.4-tetrahydro-9-carbazol-propanoylsäure oder die Verbindung 2-[4-[4-(2-Pyrimidinyl)-1-piperazinyl]butyl]-1.2-benzisothiazol-3(2H)-on-1,1-dioxid, monohydrochlorid, enthält,

ein Intensivsprengmittel aus der Gruppe quervernetzte Natriumcarboxymethylcellulose oder Natriumstärkeglycolat (sodium starch glycolat; NF XVI), als Netzmittel Natriumlaurylsulfat und als Bindemittel PVP-25 enthält, und

b) einer freisetzungskontrollierenden Doppelschicht, bestehend aus

b1) einer äußeren unverdaulichen wasserdurchlässigen Lackschicht, die im wesentlichen aus Acrylharzen auf Basis von Poly(meth)acrylsäureestern mit neutralem Charakter (NE-Typ) oder mit einem geringen Gehalt an quartären Ammoniumgruppen (R-Typ) besteht, wobei der NE-Typ aus Copoly(meth)acrylsäureestern mit dem Strukturelement

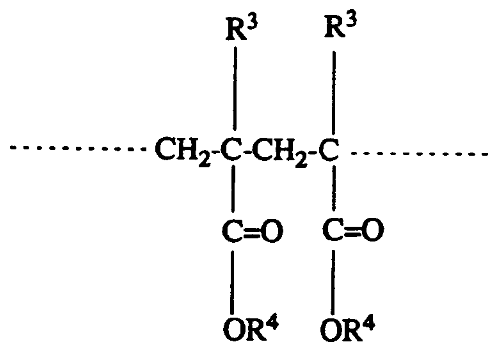

besteht, wobei

$R^3$ für H oder $CH_3$ steht, und

$R^4$ für $CH_3$ oder $C_2H_5$ steht

und ein mittleres Molekulargewicht von ca. 800.000 hat, und

der R-Typ sich hiervon dadurch unterscheidet, daß $R^4$, in einem molaren Verhältnis von 1:20 bis 1:40, für die Gruppe

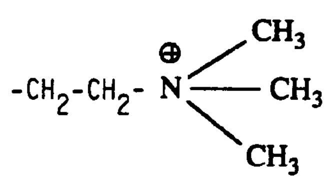

steht und ein mittleres Molekulargewicht von ca. 150.000 besitzt,

oder

aus Ethylcellulose besteht und wobei

diese äußere Lackschicht gegebenenfalls als zusätzliche Hilfsstoffe Trennmittel wie Magnesiumstearat oder Calciumstearat enthält und gegebenenfalls zusätzlich übliche Weichmacher wie Polyethylenglykol 20.000, Dialkyl-(1-4 CA)-diphthalat, Glycerintriacetat oder Zitronensäureester wie Triethylcitrat enthält, und

b2) einem inneren Mantel, der die Migration des Wassers in Richtung Kern kontrolliert, der 10 bis zu 40 % aus Hydroxypropylcellulose vom Typ M oder H besteht (HPC-M oder HPC-H) und zu 60 % bis 90 % aus einem hydrophoben Zusatz, vorzugsweise Calciumstearat oder hydriertem Rinzinusöl besteht.

2. Arzneimittel gemäß Anspruch 1, dadurch gekennzeichnet, daß der Kern das Intensivsprengmittel quervernetzte Natriumcarboxymethylcellulose enthält, die Lackschicht Polymethacrylsäurederivate der Typen RS und NE30D enthält und der Mantel als hydrophoben Zusatz Calciumstearat enthält.

3. Arzneimittel gemäß Ansprüchen 1 und 2, dadurch gekennzeichnet, daß die Pellets einen Teilchendurchmesser von 1,0 bis 3,0 mm besitzen.

4. Arzneimittel gemäß Ansprüchen 1 bis 3, dadurch gekennzeichnet, daß der Gehalt des Wirkstoffs im Kern 40 bis 90 % bezogen auf das Kerngewicht beträgt.

5. Arzneimittel gemäß Ansprüchen 1 bis 4, dadurch gekennzeichnet, daß die Lackschicht eine Dicke von bis zu 0,3 mm beträgt und ihr Gewicht bis zu 50 % des Gesamtgewichts des Pellets ausmacht.

6. Arzneimittel gemäß Ansprüchen 1 bis 5, dadurch gekennzeichnet, daß die kontrollierende Mantelschicht eine Dicke von ca. 0,1 bis 0,5 mm beträgt.

7. Arzneimittel gemäß Ansprüchen 1 bis 6, dadurch gekennzeichnet, daß die kontrollierende Mantelschicht ca. 10 bis 40 % Hydroxypropylcellulose und ca. 60 bis 90 % aus dem hydrophoben Zusatz besteht.

8. Arzneimittel gemäß Ansprüchen 1 bis 7, dadurch gekennzeichnet, daß sie einen Wirkstoff aus der Gruppe Nifedipin, Nimodipin, Nisoldipin und 4-(2-Benzyloxy-phenyl)-1,4-dihydro-2,6-dimethyl-pyridin-3-carbonsäuremethylester-5-carbonsäurecyclopropylamid sowohl in Racemform als auch als Enantiomer und (3-(4-Fluorphenylsulfonamido)-1,2,3,4-tetrahydro-9-carbazol-propanoylsäure und 2-[4-[4-(2-pyrimidinyl)-1-piperazinyl]butyl]-1,2-benzisothiazol-3-(2H)-on-1.1-dioxid, monohydrochlorid enthalten.

9. Verfahren zur Herstellung von Arzneimitteln gemäß Anspruch 1, dadurch gekennzeichnet, daß man

a) den Kern herstellt, indem man den Wirkstoff, das Intensivsprengmittel, das Netzmittel und das Bindemittel mischt und dann unter Zugabe von Wasser und/oder organischen Lösungsmitteln wie niederen aliphatischen Alkoholen oder Aceton als Granulierflüssigkeit 0,5 bis 3 Stunden im Rotorgranulator granuliert, anschließend bei 30 bis 120°C trocknet und das erhaltene Granulat siebt, und

b) das Mantelmaterial in Pulverform unter Zugabe von Granulierflüssigkeit in diskontinuierlicher oder kontinuierlicher Weise in üblichen Apparaturen für die Rollgranulation aufträgt, und

c) die Lackbestandteile entweder gelöst in organischen Lösungsmitteln wie niedere aliphatische Alkohole, flüchtige Ketone oder halogenierte Kohlenwasserstoffe aufsprüht, oder

aus wäßriger Suspension in Lackierkesseln, rotierenden Trommeln oder Tellern oder in üblichen Coatern bei Temperaturen zwischen 30 und 100°C aufträgt und thermisch vernetzt.

**Patentansprüche für folgende Vertragsstaaten : ES,GR**

1. Verfahren zur Herstellung von Arzneimitteln mit kontrollierter Wirkstoffabgabe enthaltend mindestens ein Pellet-Kollektiv, welches aufgebaut ist aus

a) einem Kern, der als Wirkstoff eine Verbindung der allgemeinen Formel

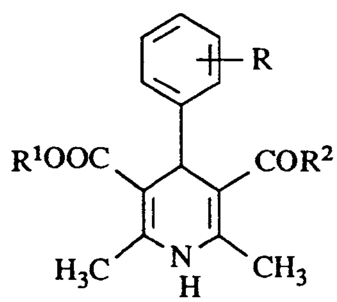

in welcher

R für Nitro oder die Gruppe

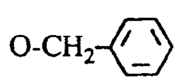

in ortho- oder meta-Position steht,

$R^1$ für Alkyl mit 1 - 4 C-Atomen steht, das gegebenenfalls durch ein Sauerstoff in der Kette unterbrochen ist und

$R^2$ für Alkoxy mit 1 - 4 C-Atomen oder für den Rest

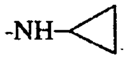

steht, oder

die Verbindung 3-(4-Fluorphenylsulfonamido ) - 1.2.3.4-tetrahydro-9-carbazol-propanoylsäure oder die Verbindung 2-[4-[4-(2-Pyrimidinyl)-1-piperazinyl]butyl]-1.2-benzisothiazol-3(2H)- on-1,1-dioxid, monohydrochlorid, enthält,

ein Intensivsprengmittel aus der Gruppe quervernetzte Natriumcarboxymethylcellulose oder Natriumstärkeglycolat (sodium starch glycolat; NF XVI), als Netzmittel Natriumlaurylsulfat und als Bindemittel PVP-25 enthält, und

b) einer freisetzungskontrollierenden Doppelschicht, bestehend aus

b1) einer äußeren unverdaulichen wasserdurchlässigen Lackschicht, die im wesentlichen aus Acrylharzen auf Basis von Poly(meth)acrylsäureestern mit neutralem Charakter (NE-Typ) oder mit einem geringen Gehalt an quartären Ammoniumgruppen (R-Typ) besteht, wobei der NE-Typ aus Copoly(meth)acrylsäureestern mit dem Strukturelement

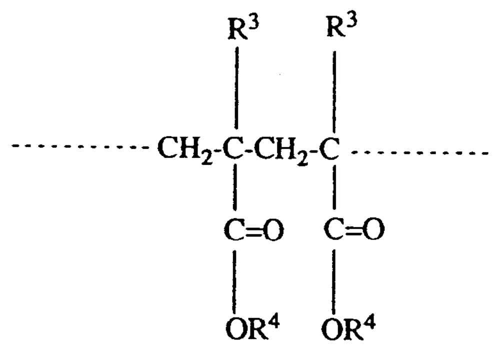

besteht, wobei

$R^3$ für H oder $CH_3$ steht, und

$R^4$ für $CH_3$ oder $C_2H_5$ steht

und ein mittleres Molekulargewicht von ca. 800.000 hat, und

der R-Typ sich hiervon dadurch unterscheidet, daß $R^4$, in einem molaren Verhältnis von 1:20 bis 1:40, für die Gruppe

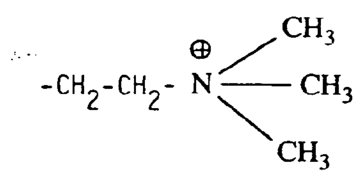

steht und ein mittleres Molekulargewicht von ca. 150.000 besitzt,

oder

aus Ethylcellulose besteht und wobei

diese äußere Lackschicht gegebenenfalls als zusätzliche Hilfsstoffe Trennmittel wie Magnesiumstearat oder Calciumstearat enthält und gegebenenfalls zusätzlich übliche Weichmacher wie Polyethylenglykol 20.000, Dialkyl-(1-4 CA)- diphthalat, Glycerintriacetat oder Zitronensäureester wie Triethylcitrat enthält, und

b2) einem inneren Mantel, der die Migration des Wassers in Richtung Kern kontrolliert, der 10 bis zu 40 % aus Hydroxypropylcellulose vom Type M oder H besteht (HPC-M oder HPC-H) und zu 60 % bis 90 % aus einem hydrophoben Zusatz, vorzugsweise Calciumstearat oder hydriertem Rizinusöl besteht,

dadurch gekennzeichnet, daß man

a) den Kern herstellt, indem man den Wirkstoff, das Intensivsprengmittel, das Netzmittel und das Bindemittel mischt und dann unter Zugabe von Wasser und/oder organischen Lösungsmitteln wie niederen aliphatischen Alkoholen oder Aceton als Granulierflüssigkeit 0,5 bis 3 Stunden im Rotorgranulator granuliert, anschließend bei 30 bis 120°C trocknet und das erhaltene Granulat siebt, und

b) das Mantelmaterial in Pulverform unter Zugabe von Granulierflüssigkeit in diskontinuierlicher oder kontinuierlicher Weise in üblichen Apparaturen für die Rollgranulation aufträgt, und

c) die Lackbestandteile entweder gelöst in organischen Lösungsmitteln wie niedere aliphatische Alkohole, flüchtige Ketone oder halogenierte Kohlenwasserstoffe aufsprüht, oder

aus wäßriger Suspension in Lackierkesseln, rotierenden Trommeln oder Tellern oder in üblichen Coatern bei Temperaturen zwischen 30 und 100°C aufträgt und thermisch vernetzt.

2. Verfahren gemäß Anspruch 1, dadurch gekennzeichnet, daß man den Kern unter Verwendung des Intensivsprengmittels quervernetzte Natriumcarboxymethylcellulose herstellt, die Lackschicht unter Verwendung von Polymethacrylsäurederivaten der Typen RS und NE30D und den Mantel unter Verwendung von Calciumstearat als hydrophobem Zusatz herstellt.

3. Verfahren gemäß Anspruch 1, dadurch gekennzeichnet, daß man in den Kern einen Wirkstoff aus der Gruppe Nifedipin, Nimodipin, Nisoldipin und 4-(2'Benzyloxy-phenyl)- 1,4-dihydro-2,6-dimethyl-pyridin-3-carbonsäuremethylester-5-carbonsäurecyclopropylamid in Racemform oder als Enantiomer oder den Wirkstoff 3-(4-Fluorphenyl-sulfonamido)- 1,2,3,4-tetrahydro-9-carbazol-propanoylsäure und 2-[4-[4-(2-pyrimidinyl)-1-piperazinyl]butyl]-1,2-benzisothiazol-3-(2H)-on-1.1-dioxid monohydrochlorid einarbeitet.

## Claims

### Claims for the following Contracting States : AT,BE,CH,DE,DK,FR,GB,IT,LI,LU,NL,SE

1. Medicaments having controlled release of the active compound and containing at least one pellet group, characterized in that the pellets are built up from

a) a core which contains as active compound a compound of the general formula

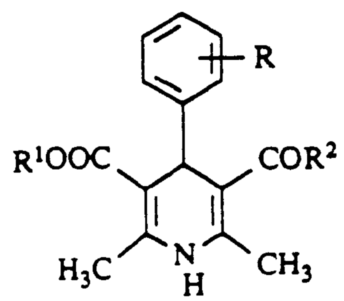

in which

R represents nitro or the group

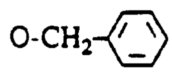

in the ortho- or meta-position,

$R^1$ represents alkyl having 1 - 4 C atoms, which is optionally interrupted by an oxygen in the chain, and

$R^2$ represents alkoxy having 1 - 4 C atoms, or represents the radical

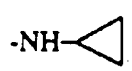

or

the compound 3-(4-fluorophenylsulphonamido)-1,2,3,4-tetrahydro-9-carbazole-propanoic acid or the compound 2-[4-[4-(2-pyrimidinyl)-1-piperazinyl]butyl]-1,2-benzoisothiazol-3(2H)-one-1,1-dioxide monohydrochloride,

and contains an intensive disintegrating agent from the group comprising crosslinked sodium carboxymethylcellulose or sodium starch glycolate (NF XVI), sodium laurylsulphate as a wetting agent and PVP-25 as a binder, and

b) a double layer which controls the release, consisting of

b1) an outer indigestible water-permeable lacquer layer which essentially consists of acrylic resins based on poly(meth)acrylic acid esters having a neutral character (NE type) or having a low content of quaternary ammonium groups (R type), the NE type consisting of copoly-(meth)acrylic acid esters having the structural element

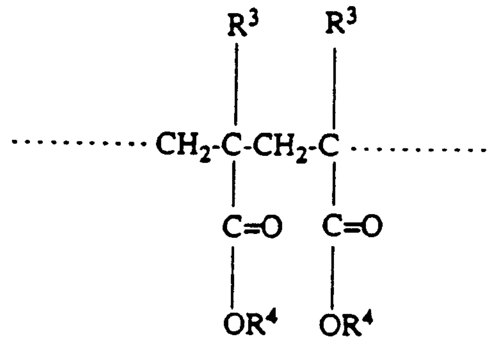

wherein
$R^3$ represents H or $CH_3$ and
$R^4$ represents $CH_3$ or $C_2H_5$
and having an average molecular weight of about 800,000, and
the R type differing from this in that $R^4$, in a molar ratio of 1:20 to 1:40, represents the group

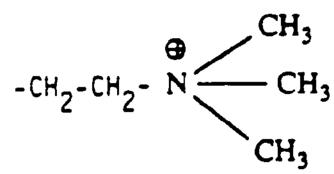

and has an average molecular weight of about 150,000,
or
consists of ethylcellulose, and
this outer lacquer layer if appropriate containing release agents, such as magnesium stearate or calcium stearate, as additional auxiliaries, and if appropriate additionally containing conventional plasticizers, such as polyethylene glycol 20,000 dialkyl (1-4 C atoms) diphthalate, glycerol triacetate or citric acid esters, such as triethyl citrate, and
b2) an inner jacket which controls the migration of the water in the direction of the core and consists of 10 to 40% of hydroxypropylcellulose of type M or H (HPC-M or HPC-H) and consists to the extent of 60% to 90% of a hydrophobic additive, preferably calcium stearate or hydrogenated castor oil.

2. Medicaments according to Claims 1, characterized in that the core contains the intensive disintegrating agent crosslinked sodium carboxymethylcellulose, the lacquer layer contains polymethacrylic acid derivatives of types RS and NE30D and the jacket contains calcium stearate as the hydrophobic additive.

3. Medicaments according to Claims 1 and 2, characterized in that the pellets have a particle diameter of 1.0 to 3.0 mm.

4. Medicaments according to Claims 1 to 3, characterized in that the content of active compound in the core is 40 to 90%, based on the core weight.

5. Medicaments according to Claims 1 to 4, characterized in that the lacquer layer has a thickness of up to 0.3 mm and its weight makes up as much as 50% of the total weight of the pellet.

6. Medicaments according to Claims 1 to 5, characterized in that the controlling jacket layer has a thickness of about 0.1 to 0.5 mm.

7. Medicaments according to Claims 1 to 6, characterized in that the controlling jacket layer consists of about 10 to 40% of hydroxyropylcellulose and about 60 to 90% of hydrophobic additive.

8. Medicaments according to Claims 1 to 7, characterized in that they contain an active compound from the group comprising nifedipine, nimodipine, nisoldipine and 4-(2-benzyloxy-phenyl)-1,4-dihydro-2,6-dimethyl-pyridine-(3-carboxylic acid methyl ester)-(5-carboxylic acid cyclopropylamide) either in racemic form or as an enantiomer and 3-(4-fluorophenyl-sulphonamido)-1,2,3,4-tetrahydro-9-carbazolepropanoic acid and 2-[4-[4-(2-pyrimidinyl)-1-piperazinyl]butyl]-1,2-benzoisothiazol-3-(2H)-one-1,1-dioxide monohydrochloride.

9. Process for the preparation of medicaments according to Claim 1, characterized in that
a) the core is prepared by mixing the active compound, the intensive disintegrating agent, the wetting agent and the binder, subsequently granulating the mixture in a rotor granulator for 0.5 to 3 hours with the addition of water and/or organic solvents, such as lower aliphatic alcohols or acetone, as the granulating liquid and then drying the mixture at 30 to 120°C and sieving the resulting granules, and

b) the jacket material is applied in powder form, with addition of granulating liquid, in a discontinuous or continuous manner in apparatuses customary for rolling granulation, and
c) the lacquer constituents are either sprayed on as a solution in organic solvents, such as lower aliphatic alcohols, volatile ketones or halogenated hydrocarbons, or applied from an aqueous suspension in lacquering kettles, rotating drums or plates or in customary coaters at temperatures between 30 and 100°C and are crosslinked by means of heat.

**Claims for the following Contracting States : ES,GR**

1. Process for the preparation of medicaments having controlled release of the active compound and containing at least one pellet group, which is built up from
a) a core which contains as active compound a compound of the general formula

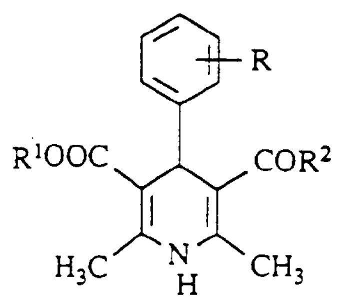

in which
R represents nitro or the group

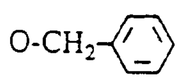

in the ortho- or meta-position,
$R^1$ represents alkyl having 1 - 4 C atoms, which is optionally interrupted by an oxygen in the chain, and
$R^2$ represents alkoxy having 1 - 4 C atoms, or represents the radical

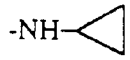 ,

or
the compound 3-(4-fluorophenylsulphonamido)-1,2,3,4-tetrahydro-9-carbazole-propanoic acid or the compound 2-[4-[4-(2-pyrimidinyl)-1-piperazinyl]butyl]-1,2-benzoisothiazol-3(2H)-one-1,1-dioxide monohydrochloride,
and contains an intensive disintegrating agent from the group comprising crosslinked sodium carboxymethylcellulose or sodium starch glycolate (NF XVI), sodium laurylsulphate as a wetting agent and PVP-25 as a binder, and
b) a double layer which controls the release, consisting of
b1) an outer indigestible water-permeable lacquer layer which essentially consists of acrylic resins based on poly(meth)acrylic acid esters having a neutral character (NE type) or having a low content of quaternary ammonium groups (R type), the NE type consisting of copoly-(meth)acrylic acid esters having the structural element

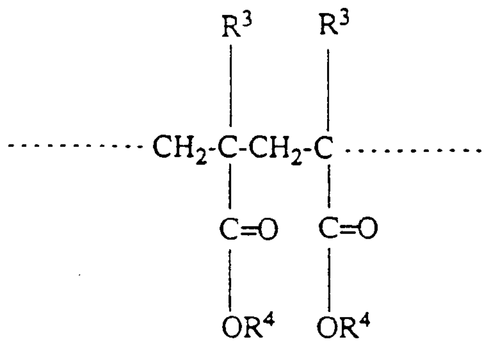

wherein
$R^3$ represents H or $CH_3$ and
$R^4$ represents $CH_3$ or $C_2H_5$
and having an average molecular weight of about 800,000, and
the R type differing from this in that $R^4$, in a molar ratio of 1:20 to 1:40, represents the group

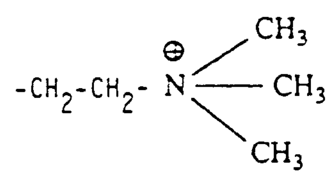

and has an average molecular weight of about 150,000,
or,
consists of ethylcellulose, and
this outer lacquer layer if appropriate containing release agents, such as magnesium stearate or calcium stearate, as additional auxiliaries, and if appropriate additionally containing conventional plasticizers, such as polyethylene glycol 20,000 dialkyl (1-4 C atoms) diphthalate, glycerol triacetate or citric acid esters, such as triethyl citrate, and
b2) an inner jacket which controls the migration of the water in the direction of the core and consists of 10 to 40% of hydroxypropylcellulose of type M or H (HPC-M or HPC-H) and consists to the extent of 60% to 90% of a hydrophobic additive, preferably calcium stearate or hydrogenated castor oil,
characterized in that
a) the core is prepared by mixing the active compound, the intensive disintegrating agent, the wetting agent and the binder, subsequently granulating the mixture in a rotor granulator for 0.5 to 3 hours with the addition of water and/or organic solvents, such as lower aliphatic alcohols or acetone, as the granulating liquid and then drying the mixture at 30 to 120°C and sieving the resulting granules, and
b) the jacket material is applied in powder form, with addition of granulating liquid, in a discontinuous or continuous manner in apparatuses customary for rolling granulation, and
c) the lacquer constituents are either sprayed on as a solution in organic solvents, such as lower aliphatic alcohols, volatile ketones or halogenated hydrocarbons, or
applied from an aqueous suspension in lacquering kettles, rotating drums or plates or in customary coaters at temperatures between 30 and 100°C and are crosslinked by means of heat.

2. Process according to Claim 1, characterized in that the core is prepared using the intensive disintegrating agent crosslinked sodium carboxymethylcellulose, the lacquer layer is prepared using polymethacrylic acid derivatives of types RS and NE30D and the jacket is prepared using calcium stearate as the hydrophobic additive.

3. Process according to Claim 1, characterized in that an active compound from the group comprising nifedipine, nimodipine, nisoldipine and 4-(2-benzyloxyphenyl)-1,4-dihydro-2,6-dimethyl-pyridine-(3-carboxylic acid methyl ester)-(5-carboxylic acid cyclopropylamide) in racemic form or as an enantiomer and the active compound 3-(4-fluorophenylsulphonamido)-1,2,3,4-tetrahydro-9-carbazole-propanoic acid and 2-[4-[4-(2-pyrimidinyl)-1-piperazinyl]butyl]-1,2-benzoisothiazol-3-(2H)-one-1,1-dioxide monohydrochloride are incorporated into the core.

## Revendications

**Revendications pour les Etats contractants suivants : AT,BE,CH,DE,DK,FR,GB,IT,LI,LU,NL,SE**

1. Médicaments à libération réglée (contrôlée) de la substance active, contenant au moins un ensemble de pastilles, médicaments caractérisés en ce que les pastilles sont constituées de :

a) un noyau qui contient comme substance active un composé de formule générale

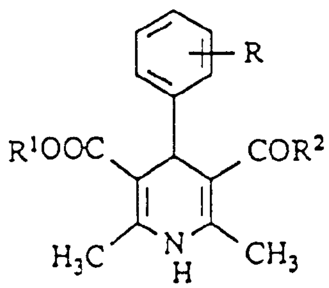

dans laquelle

R représente un groupe nitro ou le groupe

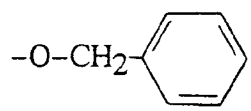 ,

en position ortho ou méta,

$R^1$ représente un groupe alkyle ayant 1 à 4 atomes de carbone, dont la chaîne peut éventuellement être interrompue par un atome d'oxygène, et

$R^2$ représente un groupe alcoxy ayant 1 à 4 atomes de carbone ou le reste

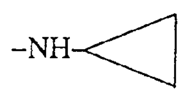 ,

ou bien

le composé acide 3-(4-fluorophénylsulfonamido)-1,2,3,4-tétrahydro-9-carbazole-propanoylique (ou propanoïque) ou le composé monochlorhydrate de 1,1-dioxyde de 2-[4-[4-(2-pyrimidinyl)-1-pypérazinyl]butyl]-1,2-benzisothiazole-3(2H)-one,

un agent de désagrégation intense, choisi parmi de la carboxyméthylcellulose sodique réticulée ou de l'amidon-glycolate de sodium ("sodium starch glycolat" ; NF XVI), comme agent de mouillage du laurylsulfate de sodium et comme liant de la "PVP-25", et

b) une double couche commandant la libération et consistant en

b1) une couche extérieure de vernis perméable à l'eau, non digestible, qui consiste essentiellement en des résines acryliques à bases d'esters de poly[acide (méth)acryliques] de caractère neutre (type NE) ou ayant une faible teneur en des groupes ammonium quaternaire (type R), le type NE consistant en des copolyesters d'acide (méth)acrylique comportant l'élément ou motif structurel :

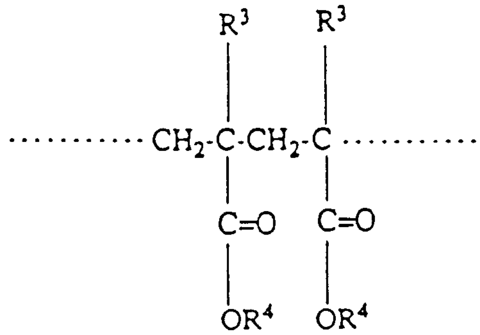

dans lequel

$R^3$ représente H ou $CH_3$, et

$R^4$ représente $CH_3$ ou $C_2H_5$,

et ayant un poids moléculaire moyen de 800,000, et

le type R s'en différenciant par le fait que $R^4$ représente, selon un rapport molaire de 1:20 à 1:40, le groupe

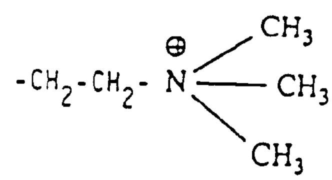

et a un poids moléculaire moyen d'environ 150 000,

ou

consiste en de l'éthylcellulose, et

la couche de vernis extérieur contient éventuellement comme additifs supplémentaires des agents de séparation comme du stéarate de magnésium ou du stéarate de calcium qui contient éventuellement des plastifiants usuels supplémentaires comme du polyéthylèneglycol 20 000, du diphtalate de dialkyle (1 à 4 atomes de carbone), du triacétate de glycérol ou un ester d'acide citrique comme le citrate de triéthyle, et b2) une enveloppe intérieure, qui régule la migration de l'eau en direction du noyau et consiste pour 10 à 40 % en de l'hydroxypropylcellulose de type M ou H (HPC-M ou HPC-H) et pour 60 à 90 % en un additif hydrophobe, avantageusement du stéarate de calcium ou de l'huile de ricin hydrogénée.

2. Médicaments selon la revendication 1, caractérisés en ce que le noyau contient comme agent de désagrégation intense de la carboxyméthylcellulose sodique réticulée, la couche de vernis contenant des dérivés d'acide polyméthacrylique de type RS et NE30D et l'enveloppe contient comme additif hydrophobe du stéarate de calcium.

3. Médicaments selon les revendications 1 et 2, caractérisés en ce que les pastilles ont un diamètre particulaire de 1,0 à 3,0 mm.

4. Médicaments selon les revendications 1 à 3, caractérisés en ce que la teneur en substance active représente dans le noyau 40 à 90 % par rapport au poids du noyau.

5. Médicaments selon les revendications 1 à 4, caractérisés en ce que la couche de vernis a une épaisseur allant jusqu'à 0,3 mm et son poids représente jusqu'à 50 % du poids total de la pastille.

6. Médicaments selon les revendications 1 à 5, caractérisés en ce que la couche d'enveloppe exerçant un rôle de réglage a une épaisseur d'environ 0,5 à 0,5 mm.

7. Médicaments selon les revendications 1 à 6, caractérisés en ce que la couche d'enveloppe exerçant un rôle de réglage consiste pour environ 10 % à 40 % en de l'hydroxypropylcellulose et pour environ 60 à 90 % en un additif hydrophobe.

8. Médicaments selon les revendications 1 à 7, caractérisés en ce qu'ils contiennent une substance active choisie parmi la nifédipine, la nimodipine et le 4-(2-benzyloxy-phényl)-1,4-dihydro-2,6-diméthyl-pyridine-3-(carboxylate de méthyle)-5-(carboxycyclopropylamide), aussi bien sous forme de racémique que sous forme d'un énantiomère, et de l'acide 3-(4-fluorophénylsulfonamido)-1,2,3,4-tétrahydro-9-carbazole-propanoylique et du monochlorhydrate de 1,1-dioxyde de 2-[4-[4-(2-pyrimidinyl)-1-pipérazinyl]butyl]-1,2-benzisothiazole-3(2H)-one.

9. Procédé pour préparer des médicament selon la revendication 1, caractérisé en ce que :

a) on prépare le noyau en mélangeant la substance active, l'agent de désagrégation intense, l'agent de mouillage et le liant, et en ajoutant ensuite de l'eau et/ou des solvants organiques comme des alcools aliphatiques inférieurs ou de l'acétone comme liquide de granulation et en granulant durant 1,5 h à 3 h dans un granulateur à rotor(s), puis en séchant à 30 jusqu'à 120°C et en tamisant le granulat obtenu, et

b) on applique la matière d'enveloppe, sous forme de poudre avec addition d'un liquide de granulation, de manière discontinue ou continue, dans des appareillages usuels pour la granulation à l'aide de rouleaux, et

c) on projette par pulvérisation les constituants du vernis, dans des solvants organiques comme des alcools aliphatiques inférieurs, des cétones volatiles ou des hydrocarbures halogénés, ou bien
on applique les constituants du vernis en suspension aqueuse dans des récipients pour vernissage, des tambours rotatifs ou des plateaux ou disques ou dans des appareils usuels pour revêtements, à des températures comprises entre 30 et 100°C, et l'on effectue une réticulation par voie thermique.

**Revendications pour les Etats contractants suivants : ES,GR**

1. Procédé pour préparer des médicaments présentant une libération réglée de la substance active et contenant au moins un ensemble de pastilles, qui est formé :

a) d'un noyau contenant comme substance active un composé de formule générale

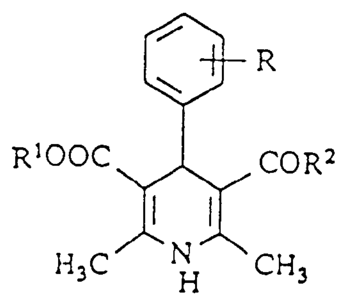

dans laquelle

R représente un groupe nitro ou le groupe

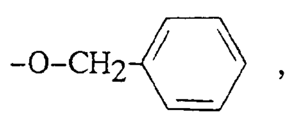

en position ortho ou méta,

$R^1$ représente un groupe alkyle ayant 1 à 4 atomes de carbone, dont la chaîne peut éventuellement être interrompue par un atome d'oxygène, et

$R^2$ représente un groupe alcoxy ayant 1 à 4 atomes de carbone ou le reste

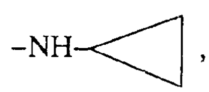

ou bien

le composé acide 3-(4-fluorophénylsulfonamido)-1,2,3,4-tétrahydro-9-carbazole-propanoylique ou le composé mono-chlorhydrate de 1,1-dioxyde de 2-[4-[4-(2-pyrimidinyl)-1-pypérazinyl]-butyl]-1,2-benzisothiazole-3(2H)-one,

un agent de désagrégation intense, choisi parmi de la carboxyméthylcellulose sodique réticulée ou de l'amidon-glycolate de sodium ("sodium starch glycolat" ; NF XVI), comme agent de mouillage du laurylsulfate de sodium et comme liant de la "PVP-25", et

b) une double couche commandant la libération et consistant en

b1) une couche extérieure de vernis perméable à l'eau, non digestible, et qui consiste essentiellement en des résines acryliques à base de poly[esters d'acide (méth)acryliques] de caractère neutre (type NE) ou

ayant une faible teneur en des groupes ammonium quaternaire (type R), le type NE consistant en des copolyesters d'acide (méth)acrylique comportant l'élément ou motif de structure :

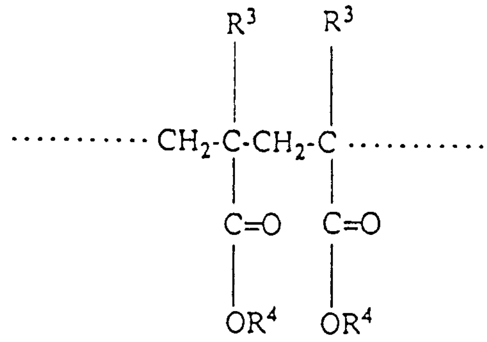

dans lequel

$R^3$ représente H ou $CH_3$, et

$R^4$ représente $CH_3$ ou $C_2H_5$,

et ayant un poids moléculaire moyen d'environ 800,000, et

le type R s'en différenciant par le fait que $R^4$ représente, selon un rapport molaire de 1:20 à 1:40, le groupe

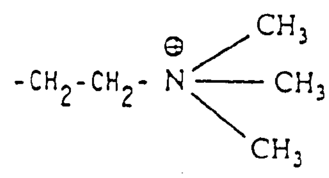

et a un poids moléculaire moyen d'environ 150 000,

ou

consiste en de l'éthylcellulose, et

la couche de vernis extérieur contient éventuellement, à titre d'adjuvants ou additifs supplémentaires, des agents de séparation comme du stéarate de magnésium ou du stéarate de calcium qui contient éventuellement des plastifiants usuels supplémentaires comme du polyéthylèneglycol 20 000, du diphtalate de dialkyle (1 à 4 atomes de carbone), du triacétate de glycérol ou des esters de l'acide citrique comme le citrate de triéthyle, et

b2) une enveloppe intérieure, qui règle ou "contrôle" la migration de l'eau en direction du noyau et consiste pour 10 à 40 % en de l'hydroxypropylcellulose de type M ou H (HPC-M ou HPC-H) et pour 60 à 90 % en un additif hydrophobe comme le stéarate de calcium ou de l'huile de ricin hydrogénée,

procédé caractérisé en ce que :

a) on produit le noyau en mélangeant la substance active, l'agent de désagrégation intense, l'agent de mouillage et le liant, puis en granulant durant 1/2 h à 3 h dans un granulateur, avec addition d'eau et/ou de solvants organiques comme des alcools aliphatiques inférieurs ou l'acétone comme liquide de granulation, puis en séchant entre 30 et 120°C et en tamisant le granulat ainsi obtenu, et

b) on applique la matière d'enveloppe, sous forme pulvérulente, avec addition d'un liquide de granulation, de façon discontinue ou continue, dans des appareillages usuels pour la granulation à l'aide de rouleaux, et

c) on projette par pulvérisation les constituants du vernis, dissous dans des solvants organiques comme des alcools aliphatiques inférieurs, des cétones volatiles ou des hydrocarbures halogénés, ou bien

on applique ces constituants du vernis en suspension aqueuse dans des récipients pour vernissage, des tambours rotatifs ou des plateaux ou disques ou dans des appareils usuels pour revêtements, à des températures comprises entre 30 et 100°C, et l'on soumet ensuite à réticulation thermique.

2. Procédé selon la revendication 1, caractérisé en ce qu'on produit le noyau en utilisant comme agent de désagrégation intensif de la carboxyméthylcellulose sodique réticulée, on prépare la couche de vernis en utilisant des dérivés d'un poly(acide méthacrylique) du type RS ou NE30D et l'on produit l'enveloppe en utilisant

du stéarate de calcium comme additif hydrophobe.

3. Procédé selon la revendication 1, caractérisé en ce qu'on incorpore au noyau une substance active choisie dans l'ensemble formé par la nifédipine, la nimodipine, la nisoldipine et le 4-(2 -benzyloxy-phényl)-1,4-dihydro-2,6-diméthyl-pyridine-3-(carboxylate de méthyle)-5-(carboxycyclopropylamide) sous forme racémique ou d'énantiomère, ou la substance acide 3-(4-fluorophényl-sulfonamido)- 1,2,3,4-tétrahydro-9-carbazolepropanoylique et le monochlorhydrate de 1,1-dioxyde de 2-[4-[4-(2-pyrimidinyl)-1-pipérazinyl]butyl]- 1,2-benzisothiazole-3(2H)-one.

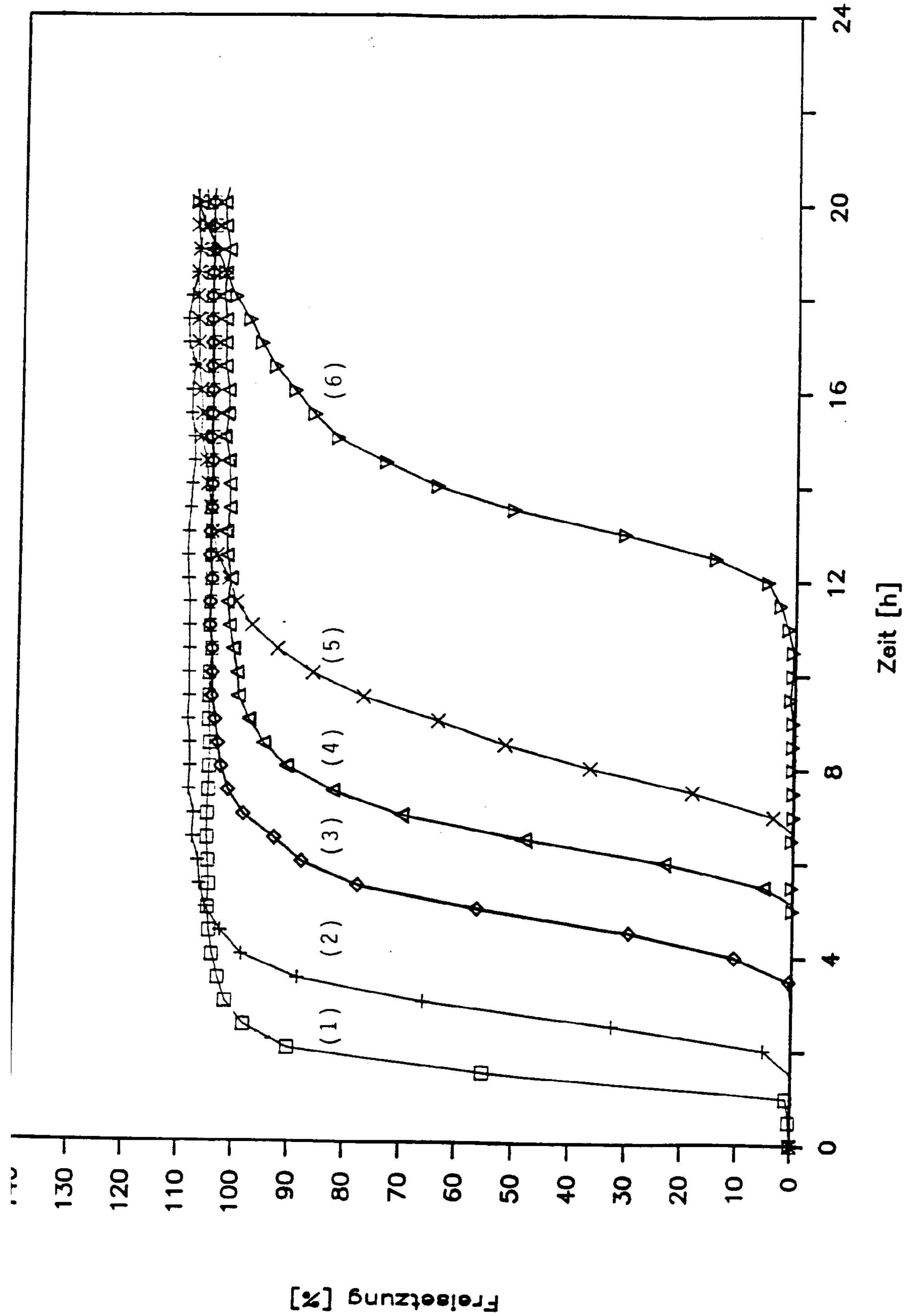
Freisetzung [%]
0
10
20
30
40
50
60
70
80
90
100
110
120
130
Zeit [h]
0
4
8
12
16
20
24
(1)
(2)
(3)
(4)
(5)
(6)

FIG.1

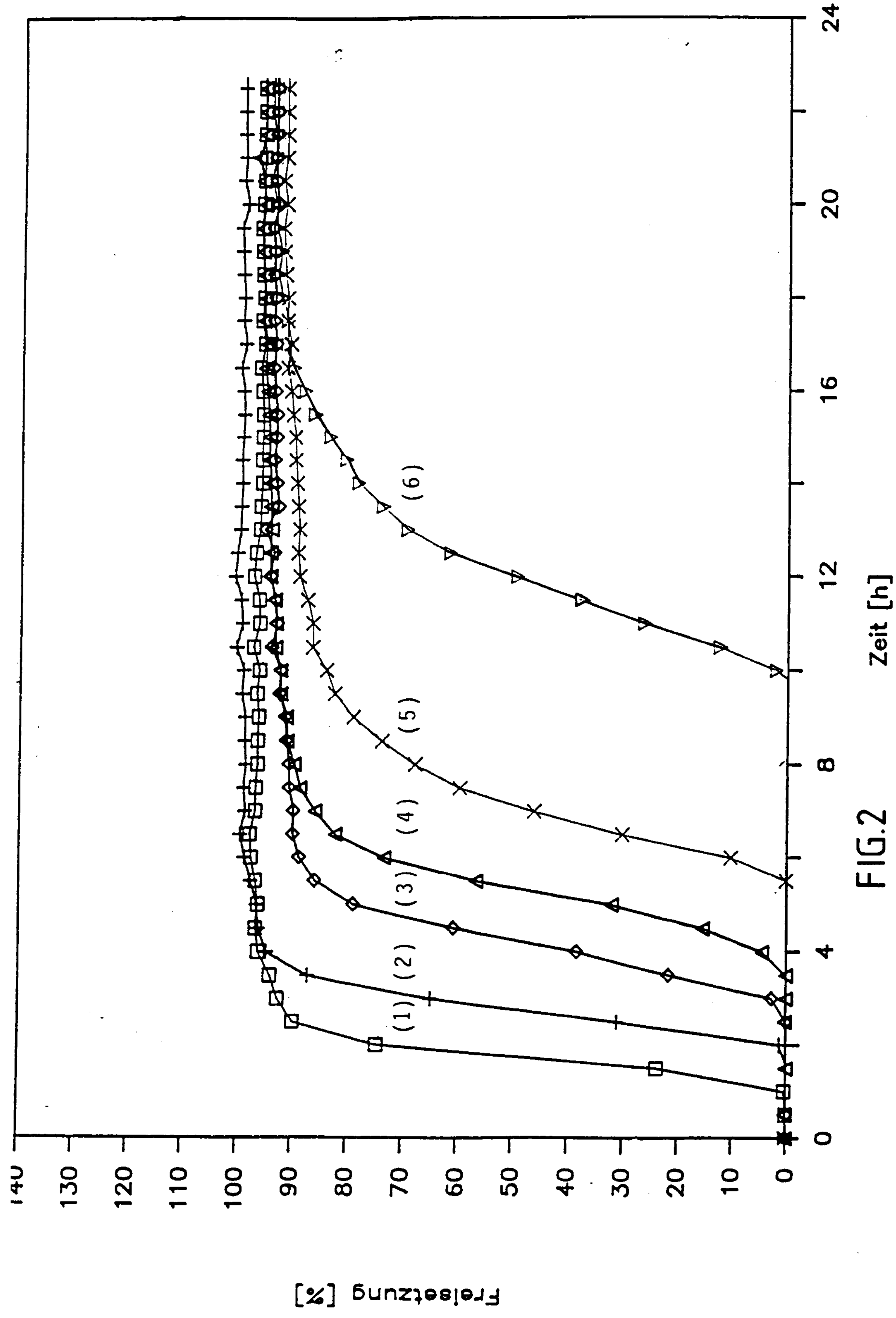
0
10
20
30
40
50
60
70
80
90
100
110
120
130
140
Freisetzung [%]
0
4
8
12
16
20
24
Zeit [h]
(1)
(2)
(3)
(4)
(5)
(6)

FIG.2